# EUROPEAN PATENT APPLICATION

(11) **EP 1 637 589 A1**
(43) Date of publication of application: **22.03.2006**
(21) Application number: 04733177.2
(22) Date of filing: 14.05.2004
(51) Int. Cl.: C12N 5/06, C12N 5/08, A61K 35/12, A61P 1/00, A61P 1/16, A61P 1/18, A61P 9/00, A61P 11/00, A61P 13/12, A61P 17/00, A61P 25/00, C12N 15/00

(54) **NOVEL STEM CELL DERIVED FROM ADULT TISSUE AND USE THEREOF**

(30) Priority: 16.05.2003 JP 2003139605; 09.10.2003 JP 2003351168; 17.12.2003 JP 2003419400
(71) Applicant: KYOWA HAKKO KOGYO CO., LTD., Chiyoda-ku, Tokyo 100-8185 (JP); Keio University, Tokyo 108-8345 (JP)
(72) Inventor: SUDA, Toshio, Tokyo 102-0093 (JP); HIRAO, Atsushi, Kanazawa-shi, Ishikawa 921-8105 (JP); ARAI, Fumio, Tokyo 135-0052 (JP); SAKURADA, Kazuhiro, Yokohama-shi, Kanagawa 225-0016 (JP); YAMADA, Yoji, c/o Kyowa Hakko Kogyo Co.,Ltd., Machida-shi, Tokyo 194-8533 (JP); ANDO, Hiroshi, c/o Kyowa Hakko Kogyo Co.,Ltd., Machida-shi, Tokyo 194-8533 (JP); SATO, Hidetaka, Kyowa Hakko Kogyo Co.,Ltd., Machida-shi, Tokyo 194-8533 (JP); YOKOYAMA, Hiromi, Kyowa Hakko Kogyo Co.,Ltd., Machida-shi, Tokyo 194-8533 (JP); ISHIHARA, Masahiko, Kyowa Hakko Kogyo Co.,Ltd., Tokyo 100-8135 (JP)
(74) Representative: Vossius & Partner
(86) International application number: PCT/JP2004/006912
(87) International publication number: WO 2004/101775

(57) **Abstract**

Subjects of the present invention are to establish a technology for separating, isolating and culturing a stem cell derived from adult tissue while retaining the *in vivo* properties thereof, and to provide a preventive and/or therapeutic agent for diseases which accompany tissue injury, which comprises a stem cell derived from adult tissue as an active ingredient.

In order to solve the subjects, the present invention provides a stem cell derived from adult tissue which is CD45-negative and CXCR4-positive. Also, the present invention provides a preventive and/or therapeutic agent for diseases which accompany tissue injury, which comprises the stem cell as an active ingredient.

## Description

### Technical Field

The present invention relates to a stem cell which is present in adult tissues such as bone marrow, skin, skeletal muscle, fat tissue and peripheral blood. The present invention also relates to a preventive and/or therapeutic agent for diseases which accompany tissue injury that comprises the stem cell, and a method for using the stem cell.

### Background Art

There are no effective therapeutic methods for completely treating diseases which accompany injury, denaturation and heteroneoformation (fibrosis, *etc.*) of tissues, such as neurodegeneration disease, cerebral infarction, obstructive vascular disease, myocardial infarction, cardiac failure, chronic obstructive lung disease, pulmonary emphysema, bronchitis, interstitial pulmonary disease, asthma, hepatitis B, hepatitis C, alcoholic hepatitis, hepatic cirrhosis, hepatic insufficiency, pancreatitis, diabetes mellitus, Crohn disease, inflammatory colitis, IgA glomerulonephritis, glomerulonephritis, renal insufficiency, decubitus, burn, sutural wound, laceration, incised wound, bite wound, dermatitis, cicatricial keloid, keloid, diabetic ulcer, arterial ulcer and venous ulcer.

The progress in stem cell biology of recent years enabled examining of techniques for inducing differentiation of human tissues and cells from a cultured stem cell for the purpose of treating the diseases. Stem cell is a cell which has self-renewal ability and also pluripotency by which it can differentiate into various tissues. Based on the collected regions, the stem cell can be classified roughly into 5 types, namely embryonic stem cell (ES cell), fetal stem cell, adult stem cell, cord blood stem cell and placental stem cell. The term adult according to the present invention means an individual after birth. That is, the adult tissues mean non-fetal and non-embryonic tissues.

Since the ES cell (embryonic stem cell) separated from a region called internal cell mass of the embryo of blastula stage and the EG cell (embryonic germ cell) collected from the gonad of fetus have totipotency of being able to differentiate into all adult cells, they drawing attention as the materials for reconstructing tissues [*Kyo no Ishyoku (Today's Transplantation),* 14, 542-548 (2001)]. Also, tissue-specific stem cells such as nerve stem cell have been separated from tissues of a fetus and cultured [*Proc. Natl. Acad Sci. USA,* 97, 14720-14725 (2000)]. However, since it is necessary to injure an embryo or a fetus for obtaining such embryo- or fetus-derived stem cell, there is an ethical problem. Also, similar to the case of organ transplantation from brain-dead patients, it is not easy to avoid a problem of immune rejection since it is not patients' own cell. In addition, since the stem cell of an embryo and the stem cell of a fetus are cells which function for the generation of individuals, their properties are different from those of the adult stem cell and their affinity for adult tissue is also different. In reality, when an ES cell is transplanted into an adult tissue, it forms tumor. These reasons show that inspection of long-term safety of a therapeutic method by transplanting an embryo- or fetus-derived cell into an adult is not easy to carry out.

In the case of an adult-derived stem cell, it is possible to carry out a treatment using the patient's own cell.

Since the stem cell is possessed of the self-renewal ability, it is possible to produce the cell in a large scale. Thus, it is possible to guarantee safety in carrying out transplantation, by proving that the stem cell cultured and produced *in vitro* has the same quality of the stem cell in the tissue. Goods for regenerating the skin or a cartilage are already on the market *[Protein, Nucleic Acid and Enzyme,* 45, 2342 -2347 (2000)].

However, since tissue-specific stem cells present in adult tissues have a limitation in terms of dividing ability, there is a disadvantage that sufficient amount of cells are difficult to be ensured. In addition, as long as a tissue-specific stem cell is used, it has no flexibility since it can be used only for the treatment of the tissue.

However, it has been revealed recently that a pluripotent stem cell having the ability to differentiate into almost all adult cells is present in an adult tissue (WO01/11011, WO01/21767, WO01/48149).

Since the cell is possessed of an immeasurably propagating self-renewal ability, it is possible to produce the cell in a large amount. In addition, unlike embryo-derived ES cell, this cell does not form tumor when transplanted into an adult tissue. It has been shown that such a pluripotent stem cell can be obtained from afterbirth human skin, skeletal muscle and bone marrow.

However, since the cell acquires the pluripotency after a long-term culture, there is a possibility that it acquired a property which is different from that in the living body, by the culturing. When a cell whose property is artificially modified by such a culturing is transplanted with a therapeutic purpose, there may be a chance of causing side effects such as malignant alteration and heteroneoformation in patients in a long term manner.

### Disclosure of the Invention

An object of the present invention is to provide a preventive and/or therapeutic agent for diseases which accompany tissue injury, which comprises an adult tissue-derived stem cell as the active ingredient.

The present invention relates to the following (1) to (14).
(1) A stem cell derived from an adult tissue which is CD45-negative and CXCR4-positive.
(2) The stem cell according to the above-described (1), wherein the adult tissue is a tissue selected from the group consisting of bone marrow, skin, skeletal muscle, fat tissue and peripheral blood.
(3) The stem cell according to the above-described (1) or (2), which is obtained by extracting cells from bone marrow by an enzyme treatment, followed by separation using an anti-CD45 antibody, an anti-CD34 antibody and an anti-Ter119 antibody.
(4) The stem cell according to the above-described (3), wherein the enzyme is collagenase.
(5) The stem cell according to any one of the above-described (1) to (4), wherein the stem cell is a pluripotent stem cell.
(6) A method for separating the stem cell according to the above-described (1) or (2), which comprises extracting cells from bone marrow by an enzyme treatment and separating stem cells using an anti-CD45 antibody, an anti-CD34 antibody and an anti-Ter119 antibody.
(7) The method according to the above-described (6), wherein the enzyme is collagenase.
(8) The method according to the above-described (6) or (7), wherein the stem cell is a pluripotent stem cell.
(9) A preventive and/or therapeutic agent for diseases which accompany tissue injury, which comprises the stem cell according to any one of the above-described (1) to (5) as an active ingredient.
(10) The preventive and/or therapeutic agent according to the above-described (9), wherein the disease which accompanies tissue injury is any one of the neural disease, respiratory organ system disease, cardiovascular disorders, hepatic disease, pancreatic disease, digestive organ system disease, renal disease and skin disease.
(11) A method for growing the stem cell according to any one of the above-described (1) to (5), which comprises culturing the cell in a medium which comprises fibronectin and is supplemented with at least one of macrophage colony-stimulating factor (M-CSF) and leukemia inhibitory factor (LIF).
(12) A method for growing the stem cell according to any one of the above-described (1) to (5), which comprises culturing the cell in a medium supplemented with macrophage colony-stimulating factor (M-CSF), leukemia inhibitory factor (LIF) and fibronectin.
(13) A method for preventing and/or treating diseases which accompany tissue injury, which comprises using the stem cell according to any one of the above-described (1) to (5).
(14) Use of the stem cell according to any one of the described-described (1) to (5) for the manufacture of a preventive and/or therapeutic agent for diseases which accompany tissue injury.

The stem cell of the present invention includes a stem cell which is derived from an adult tissue and is CD45-negative and CXCR4-positive, preferably, a stem cell which is CD45-negative and CXCR4-positive and expresses a stem cell antigen marker can be exemplified.

The stem cell antigen marker includes CD34, c-kit, Sca-1 and the like.

As the adult tissue, there is no particular limitation, so long as it is a tissue in an adult, but examples include bone marrow, skin, skeletal muscle, fat tissue, peripheral blood and the like.

The stem cell of the present invention can be prepared by taking cells out from bone marrow by an enzyme treatment and then using antibodies for various cell markers such as an anti-CD45 antibody, an anti-CD34 antibody and an anti-Ter119 antibody. The enzyme includes trypsin, dispase, collagenase and the like.

For example, the method 1 described below can be cited as the method for extracting cells from bone marrow by a collagenase treatment.

In the present invention, the diseases which accompany tissue injury include neural diseases, respiratory organ system diseases, cardiovascular disorders, hepatic diseases, pancreatic diseases, digestive organ system diseases, renal diseases, skin diseases, lung diseases and the like.

The neural diseases include cerebral infarction, cerebrovascular accidents, Parkinson's disease, Alzheimer disease, Huntington's chorea, spinal cord injury, depression, manic-depression psychosis and the like.

The respiratory organ system diseases include chronic obstructive lung disease, pulmonary emphysema, bronchitis, asthma, interstitial pneumonia, pulmonary fibrosis and the like.

The cardiovascular disorders include obstructive vascular disease, myocardial infarction, cardiac failure, coronary artery disease and the like.

The hepatic diseases include hepatitis B, hepatitis C, alcoholic hepatitis, hepatic cirrhosis, hepatic insufficiency and the like.

The pancreatic diseases include diabetes mellitus, pancreatitis and the like.

The digestive organ system diseases include Crohn disease, ulcerative colitis and the like.

The renal diseases include IgA glomerulonephritis, glomerulonephritis, renal insufficiency and the like.

The skin diseases include decubitus, burn, sutural wound, laceration, incised wound, bite wound, dermatitis, cicatricial keloid, keloid, diabetic ulcer, arterial ulcer, venous ulcer and the like.

The lung diseases include emphysema, chronic bronchitis, chronic obstructive lung disease, cystic fibrosis, idiopathic interstitial pneumonia (pulmonary fibrosis), diffuse pulmonary fibrosis, tuberculosis, asthma and the like.

Methods for preparing the stem cell of the present invention are described below.

### 1. Method for separating stem cell in bone marrow

As the method for obtaining the stem cell of the present invention from human bone marrow, the following method can be exemplified.

A piece of bone is recovered from a human body and tissues such as muscle, tendon and cartilage on the surface of bone piece are removed. The piece is made into fine bone pieces by finely cutting and crushing it with scissors. The fine bone pieces are washed three times with PBS, and then suspended in a culture liquid containing an enzyme and incubated at 37°C for 2 hours. A bone marrow extracted cell (hereinafter referred to as BMEC) can be recovered by passing the culture liquid through a 40 µm microfilter. A cell having pluripotency is contained in the BMEC.

The enzyme includes collagenase, trypsin and the like, and collagenase can be preferably cited. Specifically, Collagenase type IA (manufactured by Sigma) and the like can be cited. The collagenase concentration is, for example, from 0.06 to 0.6%, preferably 0.2%.

The culture liquid includes Dulbecco's modified Eagle's medium (DMEM; manufactured by GIBCO) and the like containing the above-described enzyme and 2.4 units of dispase (manufactured by Gibco).

The method for separating the stem cell of the present invention from the BMEC includes a method which uses antibodies and flow cytometry (FACS sorter).

Bone marrow is a hematopoietic tissue. Accordingly, in order to separate hematocytes contained in the BMEC liquid, a CD45-positive and Ter-119-positive fraction (hereinafter referred to as BMEC CD45+ cell) is removed using an anti-CD45 antibody (BD Pharmingen 30-F11) and an anti-Ter-119 antibody (BD Pharmingen Ter-119), using a leukocyte marker CD45 and an erythrocyte marker Ter-119 as indexes.

Using a stem cell marker CD34 as an index and using an anti-CD34 antibody (BD Pharmingen RAM34), the remaining cell fraction is separated into a CD45-negative, Ter-119-negative and CD34-positive fraction (hereinafter referred to as BMEC CD45-/34+) and a CD45-negative, Ter-119-negative and CD34-negative fraction (hereinafter referred to as BMEC CD45-/34-). A CXCR4-positive cell is present in both of these two fractions. In order to further concentrate the CXCR4-positive cell, a CD45-negative, Ter-119-negative, CD34-positive and CXCR4-positive cell fraction (hereinafter referred to as BMEC CD45-/34+/ CXCR4+) and a CD45-negative, Ter-119-negative, CD34-negative and CXCR4-positive fraction (hereinafter referred to as BMEC CD45-/34-/CXCR4+) are separated using an anti-CXCR4 antibody (BD Pharmingen).

In order to further concentrate the stem cell in the BMEC CD45-/34- or BMEC CD45-/34+, a side-population cell (SP cell hereinafter) is collected using a nucleic acid staining reagent Hoechst 33342 which does not stain the stem cell, or the stem cell of the present invention can be fractionated by using CD31, CD144 and Flk-1 which are markers of stem cells including vascular endothelial cell and hematopoietic stem cell, as indexes and using an anti-CD31 antibody (BD Pharmingen MEC13.3), an anti-CD144 antibody (BD Pharmingen 11D4.1) and an anti-FLK-1 antibody (BD Pharmingen Avas12α1), by using CD117, Tie-2 and CD90 which are markers expressed in the hematopoietic stem cell, as indexes and using an anti-CD117 antibody (BD Pharmingen 2B8), an anti-Tie-2 antibody and an anti-CD90 antibody (BD Pharmingen 52-2.1), and by using a mesenchymal stem cell marker ALCAM-1 as an index and using an anti-ALCAM-1 antibody.

### 2. Method for separating and culturing stem cell in the skin

As the method for obtaining the stem cell of the present invention from human skin, the following method can be exemplified.

Skin tissue including epidermis and dermis is collected from the back of a human knee or the buttocks. Said skin tissue, with the inner part of the skin on the downside, is soaked in 0.6% trypsin (manufactured by Gibco)/DMEM/F-12 (manufactured by Gibco)/1% anti-biotics, anti-mycotics (manufactured by Gibco) and treated at 37°C for 30 minutes.

After turning over the skin tissue and lightly rubbing the inside with a pair of tweezers, the skin tissue is cut into about 1 mm² using scissors and centrifuged at 1,200 rpm at room temperature for 10 minutes. The supernatant is discarded, 25 ml of 0.1% trypsin/DMEM/F-12/1% anti-biotics, anti-mycotics is added to the tissue precipitate and stirred using a stirrer at 200 to 300 rpm at 37°C for 40 minutes. After confirming that the issue precipitate was sufficiently digested, this is mixed with 3 ml of FBS (JRH) and filtered through gauze (Type I, manufactured by PIP), a 100 µm nylon filter (FALCON), a 40 µm nylon filter (FALCON) in that order. After centrifuging at 1200 rpm at room temperature for 10 minutes and discarding the supernatant, the precipitate is washed by adding DMEM/F-12/1% anti-biotics, anti-mycotics and centrifuged at 1200 rpm at room temperature for 10 minutes. After discarding the supernatant, 5 ml of DMEM/F-12B-27 (Gibco)/1% anti-biotics, anti-mycotics/20 ng/ml EGF (Genzyme)/40 ng/ml FGF (Genzyme) is added, followed by culturing at 37°C in 5% CO₂ using a 60 mmφ culture dish for suspension cell (FALCON).

The suspension cell fraction containing sphere-formed cells is collected every one week after commencement of the culturing and centrifuged at 1200 rpm at room temperature for 10 minutes. The cell precipitate is disrupted using transfer pipettes (Samco SM262-1 S), and then the culturing is continued in the medium containing 50% conditioned medium. EGF and FGF are added every 2 to 3 days. In this manner, the skin-derived stem cell of the present invention is concentrated in the thus obtained sphere. The thus obtained skin stem cells of the present invention becomes CXCR4-positive.

### 3. Method for separating stem cell in skeletal muscle

As the method for obtaining stem cell from human skeletal muscle, the following method can be exemplified.

Connective tissues containing muscles such as outer lateral head of human brachial biceps muscle and leg sartorius muscle are extracted by skin cutting and then sutured. The thus obtained total muscle is made into a minced state using scissors or a surgical knife, and then suspended in DMEM (high glucose) containing 0.06% collagenase type IA (Sigma) and 10% FBS and incubated at 37°C for 2 hours. The cells separated from the minced muscle are recovered, and then the cells are recovered by centrifugation and suspended in DMEM (high glucose) containing 10% FBS. Smooth muscle extracted cell (hereinafter referred to as SMEC) can be recovered by firstly passing the suspension through a microfilter of 40 µm pore diameter and then passing through a microfilter of 20 µm pore diameter. The pluripotent stem cell is contained in the SMEC.

The stem cell can be separated from the SMEC liquid using antibodies and flow cytometry (FACS sorter). Firstly, in order to separate hematocytes contained in the SMEC liquid, a CD45-positive and Ter-119-positive fraction (hereinafter referred to as SMEC CD45+ cell) is removed using a leukocyte marker CD45 and an erythrocyte marker Ter-119 as indexes and using an anti-CD45 antibody (BD Pharmingen 30-F11) and an anti-Ter-119 antibody (BD Pharmingen Ter-119). The remaining cell fraction is separated into a CD45-negative, Ter-119-negative and CD34-positive fraction (hereinafter referred to as SMEC CD45-/34+) and a CD45-negative, Ter-119-negative and CD34-negative fraction (hereinafter referred to as SMEC CD45-/34-), using a stem cell marker CD34 as an index and using an anti-CD34 antibody (BD Pharmingen RAM34). A CXCR4-positive cell is present in both of these two fractions. In order to further concentrate the CXCR4-positive cell from the two fractions, a CD45-negative, Ter-119-negative, CD34-positive and CXCR4-positive fraction (hereinafter referred to as SMEC CD45-/34+/ CXCR4+) and a CD45-negative, Ter-119-negative, CD34-negative and CXCR4-positive fraction (hereinafter referred to as SMEC CD45-/34-/CXCR4+) are separated using an anti-CXCR4 antibody (BD Pharmingen).

In order to further concentrate the stem cell in the SMEC CD45-/34- or SMEC CD45-/34+, a side-population cell (SP cell hereinafter) is collected using a nucleic acid staining reagent Hoechst 33342 which does not stain the stem cell, or the cell can be fractionated by using CD31, CD144 and Flk-1 which are markers of stem cells including vascular endothelial cell and hematopoietic stem cell, as indexes and using an anti-CD31 antibody (BD Pharmingen MEC13.3), an anti-CD144 antibody (BD Pharmingen 11D4.1) and an anti-FLK-1 antibody (BD Pharmingen Avas12α1), by using CD117, Tie-2 and CD90 which are markers expressed in the hematopoietic stem cell, as indexes and using an anti-CD117 antibody (BD Pharmingen 2B8), an anti-Tie-2 antibody and an anti-CD90 antibody (BD, Pharmingen 52-2.1), and by using a mesenchymal system cell marker ALCAM-1 as an index and using an anti-ALCAM-1 antibody.

### 4. Method for separating stem cell in fat tissue

As the method for obtaining stem cell from human fat tissue, the following method can be exemplified.

Connective tissues mainly containing fat tissue of human thorax or abdomen are taken out by skin cutting and then sutured. The thus obtained fat tissue is made into a minced state using scissors or a surgical knife, and then suspended in DMEM (high glucose) containing 0.06% collagenase type IA (Sigma) and 10% FBS and incubated at 37°C for 2 hours. The cells separated from the minced fat tissue are recovered, and then the cells are recovered by centrifugation and suspended in DMEM (high glucose) containing 10% FBS. Adipocyte extracted cell (hereinafter referred to as ACEC) can be recovered by firstly passing the suspension through a microfilter of 40 µm pore diameter and then passing through a microfilter of 20 µm pore diameter. The pluripotent stem cell is contained in the ACEC.

The stem cell can be separated from the ACEC liquid using antibodies and flow cytometry (FACS sorter). Firstly, in order to separate hematocytes contained in the ACEC liquid, a CD45-positive and Ter-119-positive fraction (hereinafter referred to as ACEC CD45+ cell) is removed using a leukocyte marker CD45 and an erythrocyte marker Ter-119 as indexes and using an anti-CD45 antibody (BD Pharmingen 30-F11) and an anti-Ter-119 antibody (BD Pharmingen Ter-119). The remaining cell fraction is separated into a CD45-negative, Ter-119-negative and CD34-positive fraction (hereinafter referred to as ACEC CD45-/34+) and a CD45-negative, Ter-119-negative and CD34-negative fraction (hereinafter referred to as ACEC CD45-/34-), using a stem cell marker CD34 as an index and using an anti-CD34 antibody (BD Pharmingen RAM34). A CXCR4-positive cell is present in both of these two fractions. In order to further concentrate the CXCR4-positive cell from the two fractions, a CD45-negative, Ter-119-negative, CD34-positive and CXCR4-positive cell fraction (hereinafter referred to as ACEC CD45-/34+/ CXCR4+) and a CD45-negative, Ter-119-negative, CD34-negative and CXCR4-positive fraction (hereinafter referred to as ACEC CD45-/34-/CXCR4+) are separated using an anti-CXCR4 antibody (BD Pharmingen).

In order to further concentrate the stem cell in the ACEC CD45-/34- or ACEC CD45-/34+, a side-population cell (SP cell hereinafter) is collected using a nucleic acid staining reagent Hoechst 33342 which does not stain the stem cell, or the cell can be fractionated by using CD31, CD 144 and Flk-1 which are markers of stem cells including vascular endothelial cell and hematopoietic stem cell, as indexes and using an anti-CD31 antibody (BD Pharmingen MEC13.3), an anti-CD144 antibody (BD Pharmingen 11D4.1) and an anti-FLK-1 antibody (BD Pharmingen Avas12α1), by using CD117, Tie-2 and CD90 which are markers expressed in the hematopoietic stem cell, as indexes and using an anti-CD117 antibody (BD Pharmingen 2B8), an anti-Tie-2 antibody and an anti-CD90 antibody (BD Pharmingen 52-2.1), and by using a mesenchymal system cell marker ALCAM-1 as an index and using an anti-ALCAM-1 antibody.

### 5. Method for separating and culturing pluripotent stem cell in peripheral blood

As the method for obtaining stem cell from human peripheral blood, the following method can be exemplified.

Firstly, approximately 50 ml to 500 ml of blood is collected from a vein to collect cells, and mononuclear cells are recovered therefrom by the Ficoll-Hypaque method [Kanof, M.E. and Smith P.D., 1993, *Isolation of whole mononuclear cells from peripheral blood, in Current Protocols in Immunology* (J.E. Coligan, AM. Kruisbeek, D.H. Margulies, E.M. Shevack and W. Strober, ed.) pp. 7.1.1-7.1.5, John Wiley & Sons, New York]. Next, approximately 1×10⁷ to 1×10⁸ of human peripheral blood mononuclear cells are suspended using RPMI 1640 medium (Invitrogen) containing 10% fetal bovine serum (JRH Biosciences), 100 µg/ml Streptomycin and 100 units/ml Penicillin (Invitrogen) (hereinafter referred to as peripheral blood stem cell culture basal medium) and recovered by washing twice. The thus recovered cells are ré-suspended in the peripheral blood stem cell culture basal medium, inoculated at a density of 1×10⁷ cells per 100 mm dish (BD Falcon) and cultured in a 37°C incubator under a condition of 8% CO₂, and 10 hours thereafter, suspension cells are removed and adherent cells alone are obtained by pipetting. The thus obtained adherent cells are inoculated into a fibronectin (BD)-treated (5 µg/ml) tissue culture dish (BD Falcon) at a density of 5×10⁴ cells/cm² using the peripheral blood stem cell culture basal medium containing 3 nM phorbol 12-myristate 13-acetate (PMA, manufactured by Nakalai), 50 ng/ml of human macrophage colony-stimulating growth factor (hereinafter referred to as M-CSF) or 50 ng/ml of human M-CSF and 1000 units/ml of human leukemia inhibitory factor (hereinafter referred to as LIF, manufactured by Sigma) and cultured while exchanging half of the medium one in 5 to 7 days. Two or three weeks after the commencement of the culturing, pluripotent stem cells in peripheral blood (peripheral blood fibroblastic stem cells, hereinafter referred to as PBFSC) having a dipolar shape can be amplified and obtained.

### 6 Preventive and/or therapeutic agent for diseases which accompany tissue injury, using the pluripotent stem cell of the present invention as the active ingredient, and administration method thereof

The preventive and/or therapeutic agent for diseases which accompany tissue injury, which uses the pluripotent stem cell of the present invention obtained by the above-described method as the active ingredient, can use any of the stem cells derived from bone marrow, skin, skeletal muscle, fat and peripheral blood.

It is desirable to wash the stem cells of the present invention prepared by the above-described methods of 1 to 5 with saline using an apparatus such as Hemolite 2 manufactured by Hemonetics, which can perform concentration, washing and recovery treatments of cells in a closed system, and thereby to remove the antibodies, cytokines and the like used in their separation and culturing to a level of boundlessly close to 100%. The stem cells washed and concentrated in this manner can be used in preventing and/or treating diseases which accompany tissue injury, by injecting into a vein through a general drip infusion method or by directly injecting into the affected part.

As the dose of the stem cell of the present invention, it is preferable to administer from 10 to 10⁶ cells per dose, although it varies depending on the disease or state of the tissue injury.

In addition, the disease which accompanies tissue injury can be prevented and/or treated using stem cells, by mobilizing the stem cells of the present invention being present in the internal tissues into the tissue-injured part using an agent.

Since expression of stroma-derived factor-1 (hereinafter referred to as SDF-1) as a ligand of CXCR4 is increased in a region which received tissue injury, an internal tissue, for example the stem cell of the present invention mobilized from bone marrow by an agent, can be specifically accumulated into the region which received tissue injury. Thus, the stem cell of the present invention can selectively treat diseases which accompany tissue injury.

The agent which mobilizes stem cells that are present in internal tissues includes a polypeptide having G-CSF activity, retinoic acid or a retinoic acid derivative, a CXCR4 inhibitor and the like. These agents can be used or administered as a single preparation (combination drug) or as a combination of two or more preparations. When used as a combination of two or more preparations, they can be used or administered at the same time or separately at different times.

The polypeptide having G-CSF activity includes a polypeptide comprising the amino acid sequence represented by SEQ ID NO:1, a polypeptide consisting of an amino acid sequence in which one or more amino acids of the amino acid sequence represented by SEQ ID NO:1 are deleted, substituted or added and also having G-CSF activity, and the like.

Specific examples include nartograstim (trade name Neu-up, manufactured by Kyowa Hakko Kogyo), filgrastim (trade name Gran, manufactured by Sankyo; trade name Granulokine, manufactured by Hoffman-La Roche, trade name Neupogen, manufactured by Amgen), lenograstim (trade name Neutrogin, manufactured by Chugai Pharmaceutical; trade name Granocyte, manufactured by Aventis), pegfilgrastim (trade name Neulasta, manufactured by Amgen), salgramostim (trade name Leukine, manufactured by Schering), and the like.

Also, the polypeptide having G-CSF activity includes a polypeptide having a homology of preferably 60%, more preferably 80%, further preferably 90%, most preferably 95% or more, when homology of its amino acid sequence with G-CSF having the amino acid sequence represented by SEQ ID NO:1 is retrieved by BLAST (basic local alignment search tool),. Specific examples of the polypeptide in which one or more amino acid residues of the amino acid sequence represented by SEQ ID NO:1 are substituted and which has the G-CSF activity are shown in Table 1.

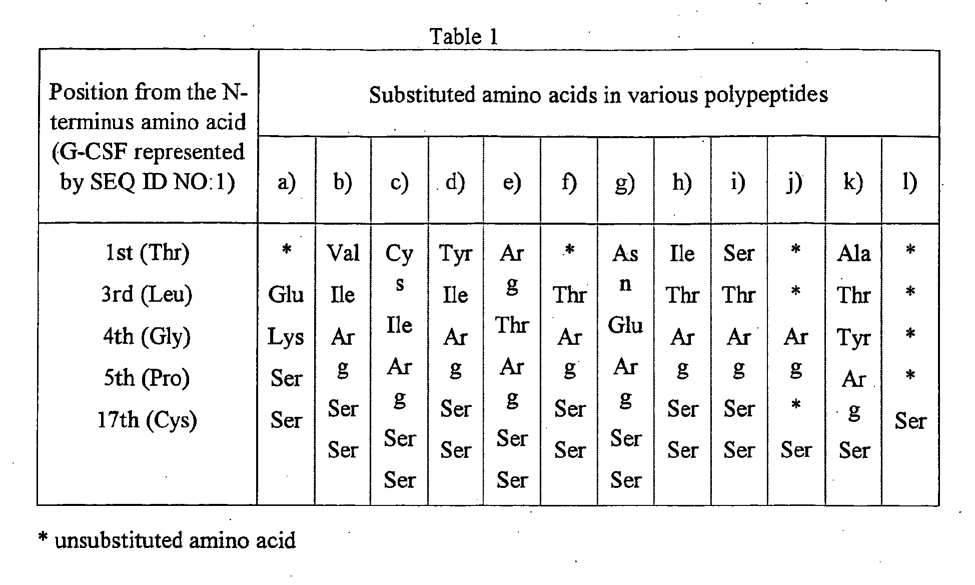

In addition, the polypeptide which has G-CSF activity may be chemically modified.

The chemical modification method includes the method described in WO 00/51626 and the like, and the polypeptides having G-CSF activity include polypeptides modified with polyalkylene glycol, such as polyethylene glycol (PEG), and having G-CSF activity.

As the retinoic acid derivative, it may be any compound which binds to a retinoic acid receptor, and examples include retinoic acid derivatives such as retinol palmitate, retinol, retinal, 3-dehydroretinoic acid, 3-dehydroretinol and 3-dehydroretinal; provitamine A such as α-carotene, β-carotene, γ-carotene, β-cryptoxanthine and echinenone; and the like. Specific examples include motretinide (trade name Tasmaderm, manufactured by Hoffman-La Roche, cf. US 4105681), compounds described in WO 02/04439, tazarotene (trade name Tazorac, manufactured by Allergan, cf. EP 284288), AGN-194310 and AGN-195183 (manufactured by Allergan, cf. WO 97/09297), retinoic acid TopiCare (trade name, Avita, manufactured by Mylan Laboratories), UAB-30 (CAS Number 205252-59-1, manufactured by UAB Research Foundation) and the like.

The CXCR4 inhibitor includes AMD-3100 and the like.

The polypeptide having G-CSF activity to be used in the present invention and retinoic acid or a retinoic acid derivative or a CXCR4 inhibitor can be used or administered as a single preparation (combination drug) or as a combination of two or more preparations, so long as they are made into pharmaceutical preparations such that they contain these respective substances as the active ingredients. When used as a combination of two or more preparations, they can be used at the same time or separately at different times. In this connection, these pharmaceutical preparations can be used, for example, in the form of tablets, capsules, granules, injections, ointments, tapes, dry powders, inhalations such as aerosols, or the like.

The above-described pharmaceutical preparations can be produced in the usual way using, in addition to the active ingredients, pharmaceutically acceptable diluents, excipients, disintegrators, lubricants, binders, surfactants, water, saline, plant oil solubilizing agents, tonicity agents, preservatives, antioxidants and the like.

In producing tablets, for example, excipients such as lactose; disintegrators such as starch, lubricants such as magnesium stearate; binders such as hydroxypropylcellulose; surfactants such as fatty acid ester; plasticizers such as glycerol; and the like can be used.

In producing injections, for example, water, saline, plant oils such as soybean oil, solvents, solubilizing agents, tonicity agents, preservatives, antioxidants and the like can be used.

In addition, inhalations are prepared using the polypeptide having G-CSF activity alone, or together with a carrier or the like which does not stimulate oral and airway mucous membranes and can facilitate their absorption by dispersing the polypeptide as minute particles. The carrier includes lactose, glycerol and the like. In addition, even in the case of these parenteral preparations, the components exemplified as additive agents for oral preparations can also be added.

The dose and administration frequency vary depending on the intended therapeutic effect, administration method, treating period, age, body weight and the like, but it is preferable to administer generally from 0.01 µg/kg to 10 mg/kg per day per adult in the case of the polypeptide having G-CSF activity and generally from 0.1 mg/kg to 100 mg/kg per day per adult in the case of retinoic acid or a retinoic acid derivative, or a CXCR4 inhibitor.

### Best Mode for Carrying Out the Invention

Examples of the present invention are shown below.

### Example 1

### Separation of bone marrow deep region stem cells from mouse femur and shinbone:

Each of 3- to 12-week-old female mice (CLEA Japan) was sacrificed by cervical spine dislocation and thoroughly disinfected with 70% ethanol, and then the skin was excised using scissors or surgical knife to obtain lower limb femur and shinbone. Under a stereoscopic microscope, tissues such as muscle, tendon and cartilage on the bones were removed using fine scissors and a surgical knife. Next, each of the bones was soaked in 0.5% trypsin-EDTA (Gibco) and incubated at 37°C for 1 hour. Further, the bone surface tissues were completely removed from the trypsin-treated bone which was then thoroughly washed with PBS. Both of the bone termini were removed using scissors, and bone marrow cells (hereinafter referred to as BMC) were recovered by pricking the needle of a syringe (filled with PBS) into the bone marrow and eluting them with sufficient amount of PBS.

Next, the eluted bone was made into bone pieces by finely cutting or crushing it using scissors, washed three times with PBS, suspended in DMEM (Gibco) containing 0.2% collagenase type IA (Sigma) and 2.4 units of dispase (Gibco), and incubated at 37°C for 2 hours. The suspension was passed through a 40 µm microfilter to obtain mouse BMEC.

The cells were washed by respectively suspending the BMC liquid and BMEC liquid in DMEM containing 5% FBS, followed by centrifugation, and suspending them again in the FBS-containing DMEM. This washing of cells was carried out twice.

In order to obtain stem cells from the BMEC liquid, they were separated using antibodies and flow cytometry (FACS sorter). Cells from the BMEC liquid were separated and recovered using propidium iodide (Molecular Probes) which stains dead cells, anti-CD45 antibody (BD Pharmingen 30-F11), anti-Ter-119 antibody (BD Pharmingen Ter-119) and anti-CD34 antibody (BD Pharmingen RAM34). Also, side-population cells (SP cells hereinafter) which are stem cell-concentrating cells and KSL cells [CD117-positive, Sca-1-positive and Linage (CD4, CD8a, CD11b, CD45R/13220, Ter-119, Gr-1, BD Pharmingen)-negative cells] as mouse hematopoietic stem cell markers were analyzed using a nucleic acid staining reagent Hoechst 33342 (SIGMA).

In addition, CD45-negative and Ter-119-negative cells in the BMEC were analyzed using each antibody of an anti-CD34 antibody, an anti-Sca-1 antibody (BD Pharmingen D7), an anti-CD31 antibody (BD Pharmingen MEC 13.3), an anti-CD144 antibody (BD Pharmingen 11D4.1), an anti-FLK-1 antibody (BD Pharmingen Avas 12α1), an anti-CD117 antibody (BD Pharmingen 2B8), an anti-ALCAM-1 antibody, an anti-Tie-2 antibody, an anti-CD90 antibody (BD Pharmingen 52-2.1) and an anti-CXCR4 antibody (BD Pharmingen 2B11).

While CD45-negative and Ter-119-negative cells were slight (0.03 to 0.5%) in the BMC obtained by the conventional method, they were approximately 22.6 to 197 times more (5.9 to 11.3%) in the BMEC obtained by the above-described method. In addition, while CD45-negative and CD34-positive cells were hardly present in the BMC, from 0.5 to 1.1% of them were present in the BMEC. The bone marrow-derived CD45-negative cells which were unable to be separated by the conventional method were recovered by the method of the present invention. The CD45-negative and Ter-119-negative cells in the BMEC were able to be separated into two groups: CD34-positive cells (1 to 18%) and CD34-negative cells (82 to 99%). On these two groups, antigens expressed on the cell surface were analyzed. Hereinafter, CD45-negative, Ter-119-negative and CD34-positive cells are called CD45-/CD34+, and CD45-negative, Ter-119-negative and CD34-negative cells are called CD45-/CD34-.

Results of the cell surface marker analysis of BMEC CD45-/CD34+ using FACS sorter were Sca-1+ (91.8%), CXCR4+ (31.5%), Flk-1+ (33.4%), CD117 (15.2%), Tie-2+ (57.2%), CD144+ (51.3%); CD31+ (58.9%), Lineage- (84.0%), CD90.2+ (83.4%), SP cell (1.3 to 1.35%). Results of the cell surface marker analysis of SP cells in BMEC CD45-/CD34+ were Sca-1 (100%), Tie-2+ (66%), CD144+ (69.3%), CD31+ (93.3%).

In the same manner, results of the cell surface marker analysis of BMEC CD45-/CD34- were Sca-1+ (17.6%), CXCR4+ (16.3%), Flk-1+.(3.6%), CD117 (19.9%), Tie-2+ (11.3%), CD144 (3.0%), CD31+ (4.5%), Lineage- (96.2%), CD90.2+ (6.9%), SP cell (0 to 0.07%). Results of the cell surface marker analysis of SP cells in BMEC CD45-/CD34- were Sca-1 (0%), Tie-2+ (24.0%), CD144+ (19.4%), CD31+ (0%).

Next, using RNeasy Mini Kit (QIAGEN), RNA was separated from cells of the BMEC CD45-/34+ and CD45-/CD34- obtained by FACS sorter, and cDNA was synthesized using Advantage RT-for PCR kit (Clontech). By using each DNA and primers having nucleotide sequences shown below, RT-PCR was carried out to analyze respective expression of Flk-1, Flt-1, Tie-2 and CXCR4. As a control, expression of glyceraldehyde 3-phosphate dehydrogenase (hereinafter referred to as GAPDH) was analyzed.

GAPDH primer: SEQ ID NOs:2 and 3

Flk-1 primer: SEQ ID NOs:4 and 5

Flt-1 primer: SEQ ID NOs:6 and 7

Tie-2 primer: SEQ ID NOs:8 and 9

CXCR4 primer: SEQ ID NOs:10 and 11, SEQ ID NOs:12 and 13

As a result of the RT-PCR, all of Flk-1, Flt-1, Tie-2 and CXCR4 were positive in BMEC CD45-/34+, but in the CD45-/34- cells, Flk-1 and CXCR4 were positive, and Flt-1 and Tie-2 were negative.

### Example 2

### Culturing of BMEC CD45-/CD 34+ and CD45-/CD34- cells:

In order to analyze pluripotency of the BMEC CD45-/CD34+ cells and CD45-/CD34- cells obtained by Example 1, a culturing test was carried out by the following method.

Each of the BMEC CD45-/CD34+ cells and CD45-/CD34- cells was cultured using a complete methyl cellulose medium MethocultoGFH4434V (StemCell Tech) under a condition of 10000 cells/ml. The incubation was set to 37°C, 5% CO₂. During the 10th to 14th days of the culturing, a muscle-like cell performing autonomic pulsation was detected in the BMEC CD45-/CD34+ cells. Thus, in order to examine expression of a heart muscle type troponin I (cardiac troponin I) and a skeletal muscle type troponin I (fast skeletal troponin I) by extracting DNA from the CD45-/CD34+ cells by the same method of Example 1, RT-PCR was carried out by using specific primers represented by SEQ ID NO:14 and SEQ ID NO:15; and SEQ ID N0:16 and SEQ ID NO:17, respectively.

As a result, their differentiation into skeletal muscle cells and heart muscle cells were confirmed because they were heart muscle type troponin I-positive and skeletal muscle type troponin I-positive. In addition, a fat cell which has oil drops inside the cell and is stained with Oil Red was also detected.

After culturing a mouse stroma cell OP-9 having high supporting ability for blood cells and vascular endothelial cells, each of the BMEC CD45-/CD34+ cells or CD45-/CD34- cells was co-cultured with OP-9 under a condition of DMEM containing 6000 cells/ml and 10% FBS. The incubation was set to 37°C, 5% CO₂.

Conditions of the cells on the 10th day of the culturing were observed under a microscope. Muscle-like cells (24 colonies) and a large number of fat-like cells were detected from the BMEC CD45-/CD34+ cells. Also, CD31-positive vascular endothelium-like cells (103 colonies) were confirmed by immuno-staining which used an anti-CD31 antibody.

When the cells became sub-confluent by the culturing using DMEM medium containing 10% FBS under a cell density condition of 5000 cells/cm², sub-culturing was carried out.

As a result of immuno-staining using anti-α-smooth muscle actin antibody (DAKO 1A4), smooth muscle cells were detected from the BMEC CD45-/CD34+ derived cultured cells after sub-culturing of 3 or more times.

Using DMEM/F12 (Gibco) containing B27 supplement (Gibco) or a medium prepared by adding 20 ng/ml of human FGF (PeproTech) and 20 ng/ml of murin EGF (PeproTech) to NeuroCulto (StemCell Tech), as a serum-free medium, 20000 cells/ml of BMEC CD45-/34+ cells or CD45-/34- cells were cultured.

As a result of FACS analysis of the CD45-/CD34- cells after culturing for about 3 weeks, 10 to 13% of CD45-/CD34+ were detected. This result shows that CD45-/CD34+ was induced and proliferated by culturing BMEC CD45-/CD34- cells.

Using a medium for nerve stem cell or a medium for nerve cell differentiation induction prepared by adding FGF and EGF, or 50 ng/ml of human β-NGF (R & D), human neurotrophin-3 (SIGMA) and human brain-derived neurotrophic factor (SIGMA), to the same B27 supplement-containing DMEM/F12, the BMEC CD45-/CD34+ cells and CD45-/CD34- cells were respectively cultured for 7 to 14 days on a laminin-coated dish (BD Biosciences) to find out, as a result, that all of the cells were able to proliferate and induce differentiation of nerve-like cells having dendrites.

Using a medium prepared by adding 50 ng/ml of human HGF (SIGMA) and 20 ng/ml of mouse EGF to HBM (Clonetics) containing 10% FBS, as a hepatocyte differentiation induction medium, 20000 cells/ml of the BMEC CD45-/CD34+ cells or CD45-/CD34- cells were respectively cultured using the laminin-coated dish (BD Biosciences). As a result, adhesion type cells were proliferated in both groups of CD45-/CD34+ cells and CD45-/CD34- cells, and cells having 2 nuclei characteristic to hepatocyte were detected.

### Example 3

### Separation and culturing of pluripotent stem cell from mouse skin thereof:

Hair of the body of a 12-week-old C57BL/6 female mouse (SLC Japan) was removed, and skin tissues were collected from its abdominal side and dorsal side. After washing with PBS (phosphate buffered saline) (GIBCO), subcutaneous tissues were physically removed, and the remained skin tissues (epidermal tissue and dermal tissue) were soaked in a 0.6% trypsin solution and allowed to undergo the reaction at 37°C for 30 minutes. The 0.6% trypsin solution was prepared by diluting a 2.5% trypsin solution (manufactured by GIBCO) with DMEM/F12 medium (manufactured by GIBCO) containing 1% antibiotic-antimyotic (manufactured by GIBCO).

After finely cutting the skin tissue into about 1 mm² using scissors, the skin tissue pieces were recovered by centrifuging (CENTRIFUGE 05P-021) (manufactured by HITACHI) at 1200 rpm for 10 minutes at room temperature, suspended in a 0.1% trypsin solution and stirred at 200 to 300 rpm for 40 minutes at 37°C using a stirrer (MAGNETIC STIRRER HS-3E) (manufactured by IUCHI). The 0.1% trypsin solution was prepared by diluting a 2.5% trypsin solution with DMEM/F12 medium containing 1% antibiotic-antimyotic.

After confirming that the skin tissue pieces were sufficiently digested, this was mixed with 1/10 volume of FBS (fetal bovine serum) (manufactured by JRH) and then filtered through gauze (Type I, manufactured by PIP), a 100 µm nylon filter (manufactured by FALCON), a 40 µm nylon filter (manufactured by FALCON) in that order. After centrifuging at 1200 rpm for 10 minutes at room temperature and discarding the supernatant, the precipitate was washed using DMEM/F-12 medium containing 1% antibiotic-antimycotic and centrifuged at 1200 rpm for 10 minutes at room temperature. The supernatant was discarded, and the residue was suspended in 5 ml of DMEM/F-12 medium containing B-27 (manufactured by GIBCO), 20 ng/ml EGF (manufactured by Genzyme); 40 ng/ml FGF (manufactured by Genzyme) and 1% antibiotic-antimycotic and cultured at 37°C in the presence of 5% CO₂ using a 60 mm diameter culture dish for suspension cell (manufactured by FALCON).

The suspension cell fraction containing sphere-formed cells were recovered every one week after commencement of the culturing, and when sub-culturing was repeated by keeping the density at 0.1× to 4x10⁶ cells/culture dish, cells which actively propagate by forming spheres (hereinafter referred to as A163 cell) were obtained. In addition, a total of 8 clones of single cell-derived cloned cell lines (hereinafter Y164, Y165, Y166, Y168, Y170, Y171, Y172 and Y173) were established from the A163 cell using limiting dilution analysis

The limiting dilution analysis was carried out by the method described below. The A163 cells of logarithmic growth phase were diluted with culture supernatant, inoculated in 0.5 cell/well portions into a 96 well plate (manufactured by IWAKI) and cultured at 37°C in the presence of 5% CO₂. EGF and FGF were added every 2 to 3 days, and 1/2 volume of the medium was exchanged every 1 week. They were extension-cultured to a 24 well plate (manufactured by IWAKI) when the number of cells reached 1000 cells/well, and A 163 single cell-derived cloned cell lines were obtained by extension culturing while keeping a cell density of 2×10⁴ cells/ml or more.

When the A163 cell made into a single cell by a trypsin solution treatment was cultured on a poly-L-ornithine (manufactured by SIGMA)/laminin (manufactured by Becton Dickinson) coat and its differentiation was induced using DMEM/F-12 medium containing 1% FBS and 1% antibiotic-antimycotic, its differentiation into an Oil Red staining-positive fat cell was observed at an efficiency of 90% or more. In addition, when its differentiation was induced using DMEM/F-12 medium containing 10% FBS and 1% antibiotic-antimycotic, its differentiation into a fibroblast, which showed a fibrous shape of 50 to 100 µm in length and clear nuclear membrane structure and was anti-fibronectin antibody (FN-3E2) (manufactured by SIGMA) positive, was observed at an efficiency of 90% or more. It was confirmed that an anti-α-smooth muscle actin antibody (1A4) (manufactured by SIGMA) positive smooth muscle cell was contained therein at a low frequency (<5%). When the same analysis was carried out on a total of 8 cloned cell lines (Y164, Y165, Y166, Y168, Y170, Y171, Y172 and Y173) established from the A163 cell, EGF-and FGF-dependent sphere forming ability and growth was observed in all clones, and differentiation into fat cell, fibroblast and smooth muscle cell was found except for Y171 and Y173. Y171 and Y173 showed the ability to differentiate into fat cell and fibroblast.

From the above results, it was revealed that the A163 cell is an adult pluripotent cell having an EGF- and FGF-dependent self-renewal ability and pluripotency.

Antibodies were stained by the following method. Cells were fixed with PBS containing 4% p-formaldehyde at room temperature for 15 minutes, washed 3 times using PBS, and then allowed to react with PBS containing 0.3% Triton X-100 (manufactured by Nakalai) at room temperature for 15 minutes. After washing 3 times with PBS, they were allowed to react with normal swine serum (manufactured by DAKO), which had been diluted to 1/20 using PBS, at room temperature for 30 minutes, and then allowed to react with an anti-fibronectin antibody diluted to 1/400 using PBS or an anti α-smooth muscle actin antibody solution (anti α-smooth muscle actin immunohistology kit) (manufactured by SIGMA) at room temperature for 1 hour. After washing 3 times with PBS, positive cells were detected using LSAB2 kit (manufactured by DAKO).

In addition, surface antigens of un-differentiated A163 cell cultured using an EGF- and FGF-added medium and A163 cell differentiation-induced into fibroblast using a 10% FBS medium were analyzed using FACS Caliber (manufactured by Becton Dickinson). The surface antigen was analyzed by the following method. After making them into single cells by a trypsin solution treatment, 5×10⁵ of the cells were suspended in PBS containing 100 µl of 0.5% BSA and allowed to undergo the reaction on ice for 45 minutes by adding from 2 to 10 µl of a primary antibody. They were washed by adding 2 ml of PBS containing 0.5% BSA, centrifuged at 1000 rpm for 5 minutes at room temperature, suspended in 1 ml of PBS and filtered through a nylon mesh (352235+ tube manufactured by FALCON), and then the measurement was carried out. When a secondary antibody was required, the reaction and washing were carried out by the same method as the primary antibody reaction after removal of the supernatant. The measurement was carried out using FACS Caliber (manufactured by Becton Dickinson) and analyzed using Cell Quest (manufactured by Becton Dickinson). As the primary antibody, an anti-CD10 rabbit antibody (manufactured by Santa Cruz), an FITC-labeled anti-CD34 antibody (manufactured by Becton Dickinson), a biotin-labeled anti-CD45 antibody (manufactured by Becton Dickinson), an FITC-labeled anti-CD90 antibody (manufactured by Becton Dickinson), a PE-labeled anti-c-kit antibody (manufactured by Becton Dickinson), a biotin-labeled anti-Ter-119 antibody (manufactured by Becton Dickinson), a PE-labeled anti-Sca-1 antibody (manufactured by Becton Dickinson), a PE-labeled anti-Flk-1 antibody (manufactured by Becton Dickinson), an FITC-labeled control rat IgG (manufactured by Becton Dickinson), a PE-labeled control rat IgG (manufactured by Becton Dickinson), a biotin-labeled rat Ig (manufactured by Becton Dickinson) and a rabbit IgG fraction (manufactured by DAKO) were used. As the secondary antibody, an FITC-labeled anti-rabbit Ig antibody (manufactured by Becton Dickinson) and an FITC-labeled streptoavidin (manufactured by Becton Dickinson) were used.

The un-differentiated A163 cell was CD10-negative, CD34-positive, CD45-negative, CD90-positive/negative, CD117-weak positive, Ter-119-negative, Sca-1-strong positive and Flk-1-weak positive, and expression of stem cell antigens CD34, c-kit and Sca-1 was confirmed. On the other hand, the A163 cell which induced differentiation into fibroblast were CD10-negative, CD34-negative, CD45-negative, CD90-positive, CD117-negative, Ter-119-negative, Sca-1-positive and Flk-1-negative, and expression disappearance of CD34 and c-kit and expression reduction of Sca-1 were observed. It was revealed, also from the expression of stem cell antigens in the un-differentiated A163 cell and the disappearance of stem cell antigens by differentiation induction, that the A163 cell is an adult skin tissue-derived pluripotent stem cell line.

### Example 4

### Expression of CXCR4 receptor in mouse skin-derived pluripotent stem cell:

Expression of CXCR4 gene of A163 cell was examined by real time RT-PCR as follows. Total RNA was obtained from the A163 cell propagating by EGF and FGF-2 and the A163 cell differentiation-induced for 3 days by a fetal bovine serum treatment using RNeasy kit (Qiagen) respectively and the genomic DNA contaminating therein was removed by treating with a DNase I (Promega). A cDNA sample was synthesized at 42°C by adding 500 ng of an oligo dT primer (Invitrogen) and a reverse transcriptase (SuperScript II, Invitrogen) to 2.5 µg of the above-described total RNA and used as the template of the PCR In the PCR, amplification was carried out in a reaction liquid containing Ex-Tag for R-PCR (TAKARA) and SYBR-Green (Biowhittaker) (40 cycles of a step of 95°C 15 seconds, 60°C 30 seconds and 70°C 30 seconds), and the amplified amount was detected by ABI PRISM 7700 Sequence detector (PERKIN ELMER). Amount of the template per one reaction corresponds to 2 ng of total RNA.

As a result of this analysis, it was found that the CXCR4 gene is expressed in the skin-derived stem cell line A163 under a condition of keeping un-differentiated state (culturing in the presence of EGF and FGF 2), and reduced at least to 1/10 by the differentiation induction with FB S stimulation.

### Example 5

### Separation and culturing of human peripheral blood pluripotent stem cell:

A cryopreservation sample of human peripheral blood mononuclear cells (5x10⁷ cells, Clonetics) was thawed at 37°C, suspended using RPMI 1640 medium (Invitrogen) containing 10% fetal bovine serum (JRH Biosciences), 100 µg/ml streptomycin and 100 units/ml penicillin (Invitrogen) and recovered by washing twice. The thus recovered cells were re-suspended in the above-described medium, inoculated at a density of 1×10⁷ cells per 100 mm dish (BD Falcon) and cultured in a 37°C incubator under a condition of 8% CO₂ and 10 hours thereafter, suspension cells were removed and adherent cells alone were obtained by pipetting. The thus obtained adherent cells were inoculated into a fibronectin (BD)-treated (5 µg/ml) or untreated tissue culture dish (BD Falcon) at a density of 5×10⁴ cells/cm² using the above-described medium containing 3 nM phorbol 12-myristate 13-acetate (PMA, manufactured by Nakalai), 50 ng/ml of human macrophage colony-stimulating growth factor (M-CSF, Sigma) or 50 ng/ml of human M-CSF and 1000 units/ml of human leukemia inhibitory factor (LIF, Sigma) and cultured while exchanging half of the medium once in 5 to 7 days. About 3 weeks after the commencement of the culturing, pluripotent stem cells having a dipolar shape appeared from the M-CSF-treated cells.

In addition, the appearing frequency was increased by the addition of LIF, and the appearing frequency was increased by culturing on the fibronectin-coated dish.

### Example 6

### Effect of G-CSF on the number of peripheral blood-derived pluripotent stem cells (PBFSC):

In accordance with Example 5, a frozen human peripheral blood mononuclear cell (hPBMC, manufactured by BioWhittarker, Cat. #CC-2702, lot #2F1388) and a G-CSF-mobilized human peripheral blood mononuclear cell (MPB, manufactured by BioWhittarker, Cat. #2G-125C, lot #2F0501) were cultured to examine effect of G-CSF to the number of the pluripotent stem cells (PBFSC).

Peripheral blood mononuclear cell-derived adherent cells were inoculated at a density of 5×10⁴ cells/cm² and cultured under fibronectin coating in the presence of M-CSF and LIF. Roughly 4 weeks thereafter, appearance of PBFSC cells from respective peripheral blood mononuclear cells was confirmed by morphology observation with the naked eye. As a result of counting the number of PBFSC per unit area, the number of appeared PBFSC was 1.1×10³ cells/cm² in the case of usual peripheral blood mononuclear cell and 2.3×10³ cells/cm² (p = 0.002) in the case of the G-CSF-mobilized peripheral blood-derived mononuclear cell, so that the number of PBFSC was significantly increased in the G-CSF-mobilized peripheral blood.

### Example 7

### Expression of nucleostemin gene in peripheral blood-derived pluripotent stem cell (PBFSC):

In order to examine whether or not stem cells are concentrated in a cell population containing PBFSC, expression of a stem cell marker nucleostemin gene was analyzed using real time RT-PCR (Tsai, RYL. *et al., Gen.* & *Dev.,* 16, 2991-3003 (2002)).

### (1) Preparation of template cDNA

Total RNA was obtained from a cell population containing PBFSC using RNeasy kit (Qiagen, Cat. #74904), and the genomic DNA contaminating therein was removed by treating with a DNase I (manufactured by Promega, Cat. #M6101). A cDNA sample was synthesized at 42°C by adding 500 ng of an oligo dT primer (manufactured by Invitrogen, Cat. #18418-012) and a reverse transcriptase (SuperScript II, manufactured by Invitrogen, Cat. #18064-014) to 2.5 µg of the above-described total RNA and used as the template of the PCR (20 µl). In preparing cDNA, a sample in which the reverse transcriptase was not added was prepared [RTase (-)].

### (2) Setting of primers

Primers for human nucleostemin gene, human GAPDH gene and human beta-actin gene were respectively set based on GenBank Acc. #AK027514, AB062273 and NM-001101 and synthesized (manufactured by Genset). Forward primer and reverse primer of the human nucleostemin gene are shown in SEQ ID NO: 18 and SEQ ID NO:19, respectively and forward primer and reverse primer of the human GAPDH gene in SEQ ID NO:20 and SEQ ID NO:21, respectively and forward primer and reverse primer of the human beta-actin gene in SEQ ID NO:22 and SEQ ID NO:23, respectively.

### (3) Confirmation of expression by RT-PCR

The PCR was carried out using ABI PRISM 7700 Sequence detector (PERKIN ELMER). The PCR was carried out by using 20 µl of a reaction solution containing 0.1 µl of the above-described cDNA (amount of the template per one reaction corresponds to 20 ng of total RNA), 300 µM for each component of dNTP (dATP, dGTP, dCTP, dTTP), 300 nM of forward and reverse primers, 1 unit of TaKaRa Ex Taq R-PCR version (TAKARA, Cat. #RR007A), 1×R-PCR buffer, 2.5 mM Mg²⁺ solution, 5% dimethyl sulfoxide (manufactured by Nakalai, Cat. #134-45) and 0.2xSYBR-Green (manufactured by Molecular Probes, Cat. #S-7567), by heating at 94°C for 5 minutes, carrying out 40 cycles of a step of 95°C 15 second, 65°C 30 seconds and 72°C 30 seconds, and then further heating at 72°C for 10 minutes. After completion of the reaction, a 10 µl portion was sampled from the thus obtained PCR reaction liquid and subjected to an electrophoresis using 2% agarose gel [prepared by dissolving AGAROSE (Nakalai) in TAE buffer (40 mM tris-acetate, 1 mM ethylenediaminetetraacetic acid)]. The gel was stained for 30 minutes with a TAE solution containing 0.5 µg/ml of ethidium bromide (Nakalai, Cat. #14631-94), and amplification of the expected DNA fragments (NS gene: 0.58 kb, GAPDH gene: 0.43 kb, beta-actin: 0.48 kb) was confirmed using a UV sample photographing device (TOYOBO FAS-III).

As a result of the analysis, in comparison with the peripheral blood-derived mononuclear cells, it was found that expression of the nucleostemin gene is increased in the PBFSC-containing cell population, and stem cells were concentrated in the PBFSC-containing cell population.

Based on the above, it was suggested that a large number of a pluripotent stem cell PBFSC capable of expressing a stem cell marker nucleostemin gene is mobilized into the peripheral blood mononuclear cells mobilized by G-CSF.

### Example 8

### Detection of bone marrow deep region stem cells from mouse femur and shinbone:

Whether or not the BMEC CD45-/CD34+ is present in the vicinity region of bone issues was observed by detecting stem cells of tissues of femur and shinbone by an immunohisto staining.

A femur and a shinbone were collected from a 5-week-old C57BL/6 mouse (CLEA Japan), soaked in a fixing liquid [4% PFA (p-formaldehyde), PBS] at 4°C for 2 hours, subsequently rinsed with PBS, soaked in a high sucrose solution [20% sucrose, PBS] and allowed to stand overnight at 4°C, and then, on the next day, embedded with an OCT (optimum cutting temperature) compound (manufactured by MILES) and frozen with isopentane which had been cooled with dry ice. The thus frozen tissue was sliced into a thickness of 4 µm using a cryostat, applied to an APS-coated slide glass (manufactured by MATSUNAMI) and thoroughly dried to prepare a frozen section.

The thus prepared frozen section was immunostained with an anti-CD34 antibody as follows.

The slide glass on which the frozen section was placed was soaked in acetone (manufactured by Wako Pure Chemical Industries) at 4°C for 10 minutes, and the slide glass was washed by soaking in PBS for 5 minutes 3 times repeatedly. The slide glass was soaked in methanol (manufactured by Wako Pure Chemical Industries) containing 0.45% hydrogen peroxide (manufactured by Wako Pure Chemical Industries) at room temperature for 30 minutes, and the slide glass was washed by soaking in PBS for 5 minutes 3 times repeatedly. This was soaked in a blocking liquid [1% BSA (manufactured by SIGMA), PBS] at room temperature for 30 minutes and then allowed to react at 4°C overnight with a solution prepared by diluting a primary antibody [biotinyl monoclonal rat anti-CD34 purified IgG, RAM 34] (manufactured by BD Biosciences) with PBS containing 1% BSA to 0.25 µg/ml. The slide glass was washed by soaking in PBS for 5 minutes 3 times repeatedly, and then soaked in a PBS solution prepared by diluting streptavidin-HPR (manufactured by Perkin Elmer) 200-fold, at room temperature for 30 minutes, and washed 3 times with PBS. Next, this was allowed to react with a biotinyl tyamide reagent (TSA Biotin System, manufactured by Perkin Elmer) at room temperature for 7 minutes, washed 5 times with PBS, and then soaked in a PBS solution prepared by diluting streptoavidin-HPR 250-fold, at room temperature for 45 minutes, and washed 3 times with PBS. This was soaked in a color developing solution [0.0075% hydrogen peroxide, 0.3 mg/ml diaminobenzidine (manufactured by Wako Pure Chemical Industries), PBS] to effect color development, washed 3 times with PBS, and then observed under a microscope ECLIPSETE 300 (manufactured by Nikon).

As a result, as CD34-positive cells, cells contacting with osteoblasts around the bones, cells directly adhered to the bone tissues and cells buried in cortical bones were observed as novel cells, in addition to the conventionally known vascular endothelial cells and partial blood cells uniformly scattered in bone marrow. This CD34+ cell in and around bone tissue is the BMEC CD45-/CD34+ cell extracted from bones after PBS elution.

### Example 9

### Transplantation of BMEC CD45-/CD34+ and CD45-/CD34- cells:

It is known that new formation of cells in intestinal epithelium, bone marrow and the like does not occur in mice when exposed to X-rays *(Nature Review Cancer,* 3, 117-129 (2003)). In this case, an X-ray-irradiated mouse was prepared particularly as a whole body disorder model, and whether or not BMEC CD45-/CD34+ and CD45-/CD34- cells are contributing to the restoration of tissues was confirmed.

In accordance with the method shown in Example 1 and using a flow cytometer, BMEC CD45-/CD34+ and CD45-/CD34- cells were separated and recovered with perfect purity from a 5-week-old mouse (C57BL/6 × 129 line) individual in which GFP gene was integrated into the whole body cells and GFP protein is being expressed therein. In addition, a femur and a shinbone were collected from an 8-week-old C57BL/6 mouse (CLEA Japan) and bone marrow cells were recovered therefrom.

The thus recovered BMEC CD45-/CD34+ cells (or CD45-/CD34- cells) were transplanted by injecting them into the caudal vein. Firstly, an 8-week-old C57BL/6 mouse (CLEA Japan) which receives the transplantation was prepared and, on the day before the transplantation, it was exposed to X-rays having a dose of 9.5 Gy using an X-ray irradiation device (manufactured by Hitachi Medico). A dose of 6,000 cells per animal of the collected BMEC CD45-/CD34+ cells, or 12,000 cells of the CD45-/CD34- cells, were respectively transplanted into the caudal vein. In order to regenerate the blood system destroyed by the X-ray irradiation, 2×10⁵ cells of bone marrow cells of the C57BL/6 mouse were simultaneously transplanted into the caudal vein. Since the GFP protein is expressed in the BMEC CD45-/CD34+ cells (or CD45-/CD34- cells), the cells which emit fluorescence in the tissues of the transplanted mouse can be detected because they are originated from the transplanted BMEC CD45-/CD34+ cells (or CD45-/CD34- cells).

By confirming that it survived after a lapse of 5 months, a transplanted mouse was prepared.

Next, said transplanted mouse was dissected to recover peripheral blood, and bone marrow cells from the leg shinbone, and then perfusion fixation was carried out to extract liver, heart, lungs, spleen, stomach, brain, skeletal muscle, skin, kidney, pancreas, small intestines and large intestine. Specifically, the transplanted mouse was anesthetized by intraperitoneal injection of Nembutal (manufactured by Dainippon Pharmaceutical), an artery of a leg was tied with a cord, and then shinbone of the leg was collected. Tips of the bone were cut off with scissors, and PBS was squeezed using 25G needle for intravenous injection manufactured by Terumo Corp. to recover bone marrow cells. Peripheral blood was recovered by exposing the heart of the mouse through ventrotomy and thoracotomy, and perfusion of the whole body was effected by inserting 25G winged needle for intravenous injection manufactured by Terumo Corp. into left ventricle, cutting right atrial appendage and flowing 20 ml of PBS. After flowing total volume of PBS, fixation was carried out by flowing 20 ml of a fixing liquid [4% PFA (p-formaldehyde), PBS] by the same operation. Thereafter, liver, heart, lungs, spleen, stomach, brain, skeletal muscle, skin, kidney, pancreas, small intestines and large intestine were extracted from the fixed mouse. The tissues other than the lungs were soaked in a fixing liquid [4% PFA (p-formaldehyde), PBS] at 4°C for 2 hours, subsequently rinsed with PBS, soaked in a high sucrose solution [20% sucrose, PBS] and allowed to stand overnight at 4°C, and then, on the next day, embedded with an OCT (optimum cutting temperature) compound (manufactured by MILES) and frozen with isopentane which had been cooled with dry ice. The lungs after extraction were injected with OCT and embedded and then frozen.

Each of the thus frozen tissues was sliced into a thickness of 10 µm using a cryostat (Frigocut 2800, manufactured by Leica), applied to an APS-coated slide glass (manufactured by MATSUNAMI) and thoroughly dried to prepare a frozen section.

The thus prepared frozen section was immunostained with an anti-GFP antibody as follows.

The slide glass on which the frozen section was placed was soaked in PBS for 5 minutes, and the slide glass was washed by repeating this step 3 times. This was soaked in a blocking liquid [5% swine serum (manufactured by DAKO), PBS] at room temperature for 1 hour and then allowed to react at room temperature for 1 hour with a solution prepared by diluting a primary antibody [polyclonal rabbit anti-GFP purified IgG, 1E4] (manufactured by MBL) 500-fold with PBS containing 1.5% swine serum. After washing 4 times with PBS, this was allowed to react at room temperature for 1 hour with a solution prepared by diluting a secondary antibody [Alexa Fluor 488-conjugated goat anti-rabbit IgG] (manufactured by Molecular Probe) 800-fold with PBS containing 1.5% swine serum. After further washing 4 times with PBS, VECTASHIELD Mounting Medium with DAPI (manufactured by VECTOR LABORATORIES) was added dropwise to the section, subsequently enclosed by covering with a cover glass and observed under a microscope Axiophot 2 manufactured by Zeiss. As a result, GFP-positive cells were observed in the tissues of the liver, lungs, spleen, brain, stomach, skin (hair follicle cell), pancreas, small intestines and large intestine of the BMEC CD45-/CD34+ transplanted mouse. Regarding the BMEC CD45-/CD34- transplanted mouse, GFP-positive cells were observed in the tissues of the spleen, kidney and small intestines.

Based on the above, it was confirmed that cells of various tissues destroyed by the X-ray irradiation were newly formed from the BMEC CD45-/CD34+ cells and CD45-/CD34- cells used in the transplantation and further adhered. Since GFP-positive cells were not observed in the peripheral blood and bone marrow cells, it was confirmed that the cells observed in the liver, lungs, spleen, brain, stomach, skin, pancreas, small intestines, large intestine and kidney were not blood system cells.

### Example 10

### Differentiation of bone marrow cells into tissue function cells in db/db mouse:

The db/db mouse is a db gene single recessive mutant mouse known as a diabetes mellitus, type II, model mouse which spontaneously generates significant diabetic symptoms such as obesity, overeating and hyperinsulinemia *(Joslin Diabetes Mellitus,* pp. 317-349 (1995)). Since obesity starts from about 4 to 5 weeks after birth of db/db mouse and its blood sugar level increases accompanied by the increase of body weight, it is considered that tissue damages such as inflammation are induced in whole body organs caused by obesity and hyperglycemia. Accordingly, examination was made on whether or not differentiation of bone marrow cells into tissue function cells is observed in db/db mouse at a higher frequency than that in normal mouse.

As the db/db mouse, a 6-week-old C57BL/KsJ-db/db female mouse (CLEA Japan) was used and, on the day before the transplantation, it was exposed to X-rays having a dose of 9.5 Gy using an X-ray irradiation device (manufactured by Hitachi Medico). On the next day, 3×10⁶ cells isolated from bone marrow of a 8-week-old C57BL/5 mouse individual, in which GFP gene was integrated into the whole body cells and GFP protein is being expressed therein, were transplanted into the caudal vein of this mouse to prepare a bone marrow cell chimeric mouse.

Four weeks after the transplantation, the mouse was dissected to carry out perfusion fixation, and respective organs were extracted. In this connection, when bone marrow cells were isolated from the femur and engrafted GFP-positive cells was analyzed using FACSCalibur (Becton Dickinson), 90% or more of the total bone marrow cells were replaced to the GFP-positive transplanted bone marrow cells. Specifically, the transplanted mouse was anesthetized by intraperitoneal injection of Nembutal (manufactured by Dainippon Pharmaceutical), the heart was exposed through ventrotomy and thoracotomy, and perfusion of the whole body was effected by inserting 25G winged needle for intravenous injection manufactured by Terumo Corp. into left ventricle, cutting right atrial appendage and flowing 20 ml of PBS. After flowing total volume of phosphate buffered saline (hereinafter referred to as PBS) to the whole body, fixation was carried out by flowing 20 ml of a fixing liquid [4% PFA (p-formaldehyde), PBS] by the same operation.

Thereafter, each organ was extracted from the fixed mouse and soaked in a fixing liquid [4% PFA (p-formaldehyde), PBS] at 4°C for 2 hours, subsequently rinsed with PBS, soaked in a high sucrose solution [20% sucrose, PBS] and allowed to stand overnight at 4°C, and then, on the next day, embedded with an OCT (optimum cutting temperature) compound (manufactured by MILES) and frozen with isopentane which had been cooled with dry ice.

The thus frozen tissue was sliced into a thickness of 10 µm using a cryostat, applied to an APS-coated slide glass (manufactured by MATSUNAMI) and thoroughly dried to prepare a frozen section. Then, an immunostaining by the following various antibodies was carried out.

The slide glass, on which the frozen section prepared by extracting the liver was placed, was washed by soaking in PBS for 5 minutes 3 times repeatedly, soaked in a blocking liquid [10% swine serum (manufactured by DAKO), PBS] at room temperature for 1 hour and then allowed to react at room temperature for 1 hour with a solution prepared by diluting a primary antibody [anti-mouse albumin rabbit polyclonal] (manufactured by Biogenesis) 200-fold with PBS containing 1.5% swine serum. After washing 4 times with PBS, this was allowed to react at room temperature for 1 hour with a solution prepared by diluting a secondary antibody [AlexaFluor 594-anti rabbit IgG] (manufactured by Molecular Probe) 800-fold with PBS containing 1.5% swine serum. After further washing 4 times with PBS, VECTASHIELD Mounting Medium with DAPI (manufactured by VECTOR LABORATORIES) was added dropwise to the section, subsequently enclosed by covering with a cover glass and observed under a fluorescence microscope. As a result, cells which are GFP-positive and albumin-positive and morphologically similar to hepatic parenchymal cells were observed.

The slide glass, on which the frozen section prepared by extracting the pancreas was placed, was washed by soaking in PBS for 5 minutes 3 times repeatedly, soaked in a blocking liquid [10% swine serum (manufactured by DAKO), PBS] at room temperature for 1 hour and then allowed to react at room temperature for 1 hour with a solution prepared by diluting a primary antibody [anti-insulin mouse monoclonal] (manufactured by Sigma) 1000-fold with PBS containing 1.5% swine serum. After washing 4 times with PBS, this was allowed to react at room temperature for 1 hour with a solution prepared by diluting a secondary antibody [AlexaFluor 594-anti mouse IgG] (manufactured by Molecular Probe) 800-fold with PBS containing 1.5% swine serum. After further washing 4 times with PBS, VECTASHIELD Mounting Medium with DAPI (manufactured by VECTOR LABORATORIES) was added dropwise to the section, subsequently enclosed by covering with a cover glass and observed under a fluorescence microscope. As a result, GFP-positive and insulin-positive pancreatic β cells were observed.

The slide glass, on which the frozen section prepared by extracting the heart was placed, was washed by soaking in PBS for 5 minutes 3 times repeatedly, soaked in a blocking liquid [10% swine serum (manufactured by DAKO), PBS] at room temperature for 1 hour and then allowed to react at room temperature for 1 hour with a solution prepared by diluting a primary antibody [anti-sarcomeric α-actinin mouse monoclonal] (manufactured by Sigma) 400-fold with PBS containing 1.5% swine serum. After washing 4 times with PBS, this was allowed to react at room temperature for 1 hour with a solution prepared by diluting a secondary antibody [AlexaFluor 594-anti mouse IgG] (manufactured by Molecular Probe) 800-fold with PBS containing 1.5% swine serum. After further washing 4 times with PBS, VECTASHIELD Mounting Medium with DAPI (manufactured by VECTOR LABORATORIES) was added dropwise to the section, subsequently enclosed by covering with a cover glass and observed under a fluorescence microscope. As a result, a tissue which is GFP-positive and actinin-positive and morphologically similar to heart muscle tissue was observed.

The slide glass, on which the frozen section prepared by extracting the small intestines was placed, was washed by soaking in PBS for 5 minutes 3 times repeatedly and then soaked in an enzyme solution [10 µg/ml proteinase K (manufactured by Gibco PBS)] at room temperature for 20 minutes. After washing twice with PBS, this was soaked in a fixing liquid [4% PFA (p-formaldehyde), PBS] for 10 minutes, soaked in a blocking liquid [10% swine serum (manufactured by DAKO), PBS] at room temperature for 1 hour and then allowed to react at room temperature for 1 hour with a solution prepared by diluting a primary antibody [anti-cytokeratin antibody mouse monoclonal] (manufactured by DAKO) 50-fold with PBS containing 1.5% swine serum. After washing 4 times with PBS, this was allowed to react at room temperature for 1 hour with a solution prepared by diluting a secondary antibody [AlexaFluor 594-anti rabbit IgG] (manufactured by Molecular Probe) 800-fold with PBS containing 1.5% swine serum. After further washing 4 times with PBS, VECTASHIELD Mounting Medium with DAPI (manufactured by VECTOR LABORATORIES) was added dropwise to the section, subsequently enclosed by covering with a cover glass and observed under a fluorescence microscope. As a result, cells which are GFP-positive and cytokeratin-positive and morphologically similar to epithelial cells were observed.

The slide glass, on which the frozen section prepared by extracting the lungs was placed, was washed by soaking in PBS for 5 minutes 3 times repeatedly and then soaked in an enzyme solution [0.25% trypsin (manufactured by Gibco), PBS] at room temperature for 2 minutes. After immediately soaking in a stopping liquid [2% fetal bovine serum (manufactured by JRH Biosciences), PBS] and washing twice with PBS, this was soaked in a fixing liquid [4% PFA (p-formaldehyde), PBS] for 4 minutes, soaked in a blocking liquid [3% bovine serum albumin (manufactured by Sigma), 0.1% Tween-20, PBS] at room temperature for 30 minutes and then allowed to react at room temperature for 1 hour with a solution prepared by diluting a primary antibody [anti-keratin antibody rabbit polyclonal] (manufactured by DAKO) 100-fold with PBS containing 1.5% swine serum. After washing 4 times with PBS, this was allowed to react at room temperature for 1 hour with a solution prepared by diluting a secondary antibody [AlexaFluor 594-anti rabbit IgG] (manufactured by Molecular Probe) 800-fold with PBS containing 1.5% swine serum. After further washing 4 times with PBS, VECTASHIELD Mounting Medium with DAPI (manufactured by VECTOR LABORATORIES) was added dropwise to the section, subsequently enclosed by covering with a cover glass and observed under a fluorescence microscope. As a result, cells which are GFP-positive and keratin-positive and morphologically similar to lung epithelial cells were observed.

The slide glass, on which the frozen section prepared by extracting the brain was placed, was washed by soaking in PBS for 5 minutes 3 times repeatedly, soaked in a blocking liquid [10% swine serum (manufactured by DAKO), 0.01% Triton, PBS] at room temperature for 1 hour and then allowed to react at room temperature for 1 hour with a solution prepared by diluting a primary antibody [anti-Neu N mouse monoclonal] (manufactured by Chemicon) 1000-fold with PBS containing 1.5% swine serum and 0.01% Triton. After washing 4 times with PBS, this was allowed to react at room temperature for 1 hour with a solution prepared by diluting a secondary antibody [AlexaFluor 594-anti mouse IgG] (manufactured by Molecular Probe) 800-fold with PBS containing 1.5% swine serum. After further washing 4 times with PBS, VECTASHIELD Mounting Medium with DAPI (manufactured by VECTOR LABORATORIES) was added dropwise to the section, subsequently enclosed by covering with a cover glass and observed under a fluorescence microscope. As a result, a GFP-positive and Neu N-positive nerve cell was confirmed. In addition, a GFP-positive cell having a dendritic shape specific to Purkinje cell was confirmed in Purkinje cells of the cerebellum tissue by a morphological observation.

Thus, it was confirmed that the bone marrow cell was newly formed into a hepatic parenchymal cell, a pancreatic β cell, a heart muscle cell, a digestive organ epithelial cell, a lung epithelial cell, a neuron and a Purkinje cell in the test to transplant bone marrow cells of a C57BL/6 mouse into an X-ray-irradiated C57BL/KsJ/db/db mouse.

Using 6-week-old female individuals of C57BL/KsJ/db/db (CLEA Japan), C57BL/KsJ/db/+m (CLEA Japan) and C57BL/6J (CLEA Japan), they were exposed to X-rays having respective doses of 9.5 Gy, 12 Gy and 9.5 Gy on the day before the transplantation using an X-ray irradiation device (manufactured by Hitachi Medico). The C57BL/KsJ/db/+m is a hetero mutation mouse of db gene which does not show diabetic symptoms such as obesity, overeating and hyperinsulinemia, so that this is used as a control of C57BL/KsJ/db/db. On the next day, 3×10⁶ of cells isolated from bone marrow of a 8-week-old C57BL/6 line mouse individual, in which GFP gene was integrated into the whole body cells and GFP protein is being expressed therein, were transplanted into the caudal vein of this mouse to prepare a bone marrow cell chimeric mouse.

Four weeks after the transplantation, the mouse was dissected to carry out perfusion fixation, and respective organs were extracted and embedded to prepare frozen sections. In this connection, when bone marrow cells were isolated from the femur and engrafted GFP-positive cells was analyzed using FACSCalibur (Becton Dickinson), 90% or more of the total bone marrow cells in the case of C57BL/KsJ/db/db, 90% or more of the total bone marrow cells in the case of C57BL/KsJ/db/+m and 60 to 80% or more of the total bone marrow cells in the case of C57BL/6J were replaced by the GFP-positive transplanted bone marrow cells. Immunostaining of the thus prepared frozen sections (20 to 40 sections) of the heart, liver and pancreas was carried out by the same method shown in (1), and bone marrow-derived heart muscle cells, hepatic parenchymal cells and pancreatic β cells were judged. The number of detected GFP-positive heart muscle cells, hepatic parenchymal cells and pancreatic β cells to the areas of analyzed tissue sections was numerically expressed as a detection frequency per analyzed tissue area [the number of GFP-positive and tissue function cells/tissue area (cells/cm²)], with the results shown in Table 2.

**Table 2**

| | | | The number of GFP-positive and tissue function cells/tissue area (cells/cm²) | | |
|---|---|---|---|---|---|
| Chimeric mice | Line | X-ray dose (* chimeric ratio) | Heart α-Actinin-positive | Liver Albumin-positive | Pancreas Insulin-positive |
| A-1 | C57BL/K sJ Db/db | 9.5 Gy (>90%) | 2.9 | 1.9 | 14.9 |
| A-2 | | | <0.1 | 1.5 | 4.9 4.9 |
| A-3 | | | <0.1 | 2.3 | 0.8 |
| D-1 | C57BL/6J | 9.5 Gy (>60 to 80%) | <0.1 | 0.071 | <0.1 |
| D-2 | | | <0.1 | 0.068 | <0.1 |
| D-3 | | | <0.1 | 0.026 | <0.1 |
| H-1 | C57BL/K sJ Db/+m | 12 Gy (>90%) | 0.8 | 0.049 | 1.8 |
| H-2 | | | <0.1 | 0.024 | 5.1 |
| H-3 | | | 0.2 | 0.023 | 25.1 |

| | | | | | |
|---|---|---|---|---|---|
| * Chimeric ratio: ratio of transplanted cells based on the total bone marrow cells | | | | | |

As shown in Table 2, it was quantitatively revealed that GFP-positive heart muscle cells, hepatic parenchymal cells and pancreatic β cells are detected at a high frequency in C57BL/KsJ/db/db mouse than in C57BL/6 mouse. Since similar degree is recognized also in C57BL/KsJ/db/+m regarding the differentiation into heart muscle cells and pancreatic β cells, the influence of gene background, namely homoplastic transplantation due to inconsistency of transplanted cell and recipient was considered as the cause. On the other hand, differentiation into hepatic parenchymal cells was average value ± standard deviation: 0.032 ± 0.015 cells/cm² in C57BL/KsJ/db/+m and average value ± standard deviation: 0.055 ± 0.025 cells/cm² in C57BL/6, which were almost the same degree, but it was average value ± standard deviation: 1.9 ± 0.4 cells/cm² in C57BL/KsJ/db/db, which was significant increase (P < 0.01), so that it was considered that function acceleration and damage of the liver induced by obesity, overeating, hyperinsulinemia and the like are the cause.

Based on the above, a phenomenon was found in which differentiation of bone marrow cells into tissue function cells and adhesion thereof are increased by the acceleration and damage of tissue functions, induced by the inconsistency of transplantation immunity system, and obesity, overeating, hyperinsulinemia and the like, in the test to transplant bone marrow cells of a C57BL/6 mouse into an X-ray-irradiated C57BL/KsJ/db/db mouse.

### Industrial Applicability

A CD45-negative and CXCR4-positive stem cell is provided by the present invention. Also, a preventive and/or therapeutic agent for diseases which accompany tissue injury, comprising said stem cell as the active ingredient, is provided by the present invention.
Free text of sequence listing
SEQ ID NO:2 - Explanation of artificial sequence: synthetic DNA
SEQ ID NO:3 - Explanation of artificial sequence: synthetic DNA
SEQ ID NO:4 - Explanation of artificial sequence: synthetic DNA
SEQ ID NO:5 - Explanation of artificial sequence: synthetic DNA
SEQ ID NO:6 - Explanation of artificial sequence: synthetic DNA
SEQ ID NO:7 - Explanation of artificial sequence: synthetic DNA
SEQ ID NO:8 - Explanation of artificial sequence: synthetic DNA
SEQ ID NO:9 - Explanation of artificial sequence: synthetic DNA
SEQ ID NO:10 - Explanation of artificial sequence: synthetic DNA
SEQ ID NO:11 - Explanation of artificial sequence: synthetic DNA
SEQ ID NO:12 - Explanation of artificial sequence: synthetic DNA
SEQ ID NO:13 - Explanation of artificial sequence: synthetic DNA
SEQ ID NO:14 - Explanation of artificial sequence: synthetic DNA
SEQ ID NO:15 - Explanation of artificial sequence: synthetic DNA
SEQ ID NO:16 - Explanation of artificial sequence: synthetic DNA
SEQ ID NO:17 - Explanation of artificial sequence: synthetic DNA
SEQ ID NO:18 - Explanation of artificial sequence: synthetic DNA
SEQ ID NO: 19 - Explanation of artificial sequence: synthetic DNA
SEQ ID NO: 20 - Explanation of artificial sequence: synthetic DNA
SEQ ID NO:21 - Explanation of artificial sequence: synthetic DNA
SEQ ID NO:22 - Explanation of artificial sequence: synthetic DNA
SEQ ID NO:23 - Explanation of artificial sequence: synthetic DNA

## Claims

1. A stem cell derived from an adult tissue which is CD45-negative and CXCR4-positive.

2. The stem cell according to claim 1, wherein the adult tissue is a tissue selected from the group consisting of bone marrow, skin, skeletal muscle, fat tissue and peripheral blood.

3. The stem cell according to claim 1 or 2, which is obtained by extracting cells from bone marrow by an enzyme treatment, followed by separation using an anti-CD45 antibody, an anti-CD34 antibody and an anti-Ter119 antibody.

4. The stem cell according to claim 3, wherein the enzyme is collagenase.

5. The stem cell according to any one of claims 1 to 4, wherein the stem cell is a pluripotent stem cell.

6. A method for separating the stem cell according to claim 1 or 2, which comprises extracting cells from bone marrow by an enzyme treatment and separating stem cells using an anti-CD45 antibody, an anti-CD34 antibody and an anti-Ter119 antibody.

7. The method according to claim 6, wherein the enzyme is collagenase.

8. The method according to claim 6 or 7, wherein the stem cell is a pluripotent stem cell.

9. A preventive and/or therapeutic agent for diseases which accompany tissue injury, which comprises the stem cell according to any one of claims 1 to 5 as an active ingredient.

10. The preventive and/or therapeutic agent according to claim 9, wherein the disease which accompanies tissue injury is any one of the neural disease, respiratory organ system disease, cardiovascular disorders,, hepatic disease, pancreatic disease, digestive organ system disease, renal disease and skin disease.

11. A method for growing the stem cell according to any one of claims 1 to 5, which comprises culturing the cell in a medium which comprises fibronectin and is supplemented with at least one of macrophage colony-stimulating factor (M-CSF) and leukemia inhibitory factor (LIF).

12. A method for growing the stem cell according to any one of claims 1 to 5, which comprises culturing the cell in a medium supplemented with macrophage colony-stimulating factor (M-CSF), leukemia inhibitory factor (LIF) and fibronectin.

13. A method for preventing and/or treating diseases which accompany tissue injury, which comprises using the stem cell according to any one of claims 1 to 5.

14. Use of the stem cell according to any one of claims 1 to 5 for the manufacture of a preventive and/or therapeutic agent for diseases which accompany tissue injury.


SEQUENCE LISTING

<110> KYOWA HAKKO KOGYO CO., LTD.

<110> KEIO UNIVERSITY

<120>

<130> 11571WO1

<150> JP2003-139605
<151> 2003-05-16

<150> JP2003-351168
<151> 2003-10-09

<150> JP2003-419400
<151> 2003-12-17

<160> 23

<170> PatentIn Ver. 2.1

<210> 1
<211> 174
<212> PRT

<213> Homo sapiens

<400> 1
Thr Pro Leu Gly Pro Ala Ser Ser Leu Pro Gln Ser Phe Leu Leu Lys
1               5                   10                  15

Cys Leu Glu Gln Val Arg Lys Ile Gln Gly Asp Gly Ala Ala Leu Gln
            20                  25                  30

Glu Lys Leu Cys Ala Thr Tyr Lys Leu Cys His Pro Glu Glu Leu Val
        35                  40                  45

Leu Leu Gly His Ser Leu Gly Ile Pro Trp Ala Pro Leu Ser Ser Cys
    50                  55                  60

Pro Ser Gln Ala Leu Gln Leu Ala Gly Cys Leu Ser Gln Leu His Ser
65                  70                  75                  80

Gly Leu Phe Leu Tyr Gln Gly Leu Leu Gln Ala Leu Glu Gly Ile Ser
                85                  90                  95

Pro Glu Leu Gly Pro Thr Leu Asp Thr Leu Gln Leu Asp Val Ala Asp
            100                 105                 110

Phe Ala Thr Thr Ile Trp Gln Gln Met Glu Glu Leu Gly Met Ala Pro
        115                 120                 125

Ala Leu Gln Pro Thr Gln Gly Ala Met Pro Ala Phe Ala Ser Ala Phe
    130                 135                 140

Gln Arg Arg Ala Gly Gly Val Leu Val Ala Ser His Leu Gln Ser Phe
145                 150                 155                 160

Leu Glu Val Ser Tyr Arg Val Leu Arg His Leu Ala Gln Pro
                165                 170

<210> 2
<211> 20
<212> DNA
<213> Artificial Sequence

<220>
<223> Description of Artificial Sequence: synthetic DNA

<400> 2
tcttcaccac catggagaag                                                20

<210> 3
<211> 20
<212> DNA
<213> Artificial Sequence

<220>
<223> Description of Artificial Sequence: synthetic DNA

<400> 3
gcttcaccac cttcttgatg 20

<210> 4
<211> 22
<212> DNA
<213> Artificial Sequence

<220>
<223> Description of Artificial Sequence: synthetic DNA

<400> 4
agaacaccaa aagagaggaa cg 22

<210> 5
<211> 22
<212> DNA
<213> Artificial Sequence

<220>
<223> Description of Artificial Sequence: synthetic DNA

<400> 5

gcacacaggc agaaaccagt ag                                                22

<210> 6
<211> 25
<212> DNA
<213> Artificial Sequence

<220>
<223> Description of Artificial Sequence: synthetic DNA

<400> 6
gcctctgatg gtgatcgtgg aatac                                             25

<210> 7
<211> 25
<212> DNA
<213> Artificial Sequence

<220>
<223> Description of Artificial Sequence: synthetic DNA

<400> 7
tctggccact tggaaactgt aggaa                                             25

<210> 8
<211> 25

<212> DNA
<213> Artificial Sequence

<220>
<223> Description of Artificial Sequence: synthetic DNA

<400> 8
ggaactctgg cccttaacag gaagg 25

<210> 9
<211> 25
<212> DNA
<213> Artificial Sequence

<220>
<223> Description of Artificial Sequence: synthetic DNA

<400> 9
gatggcaaaa gcagggtctg tctct 25

<210> 10
<211> 25
<212> DNA
<213> Artificial Sequence

<220>

<223> Description of Artificial Sequence: synthetic DNA

<400> 10
ctgcatcatc atctccaagc tgtca 25

<210> 11
<211> 25
<212> DNA
<213> Artificial Sequence

<220>
<223> Description of Artificial Sequence: synthetic DNA

<400> 11
tagaggatgg ggttcaggca acagt 25

<210> 12
<211> 21
<212> DNA
<213> Artificial Sequence

<220>
<223> Description of Artificial Sequence: synthetic DNA

<400> 12
atggaaccga tcagtgtgag t 21

<210> 13
<211> 21
<212> DNA
<213> Artificial Sequence

<220>
<223> Description of Artificial Sequence: synthetic DNA

<400> 13
ttccttggcc tttgactgtt g                                                21

<210> 14
<211> 20
<212> DNA
<213> Artificial Sequence

<220>
<223> Description of Artificial Sequence: synthetic DNA

<400> 14
ctgccaacta ccgagcctat                                                  20

<210> 15
<211> 23
<212> DNA

<213> Artificial Sequence

<220>
<223> Description of Artificial Sequence: synthetic DNA

<400> 15
gtgatgttct tggtgacttt tgc                                                23

<210> 16
<211> 22
<212> DNA
<213> Artificial Sequence

<220>
<223> Description of Artificial Sequence: synthetic DNA

<400> 16
gaagcgcaac agggccatca cg                                                 22

<210> 17
<211> 22
<212> DNA
<213> Artificial Sequence

<220>
<223> Description of Artificial Sequence: synthetic DNA

<400> 17
ccacgtcacg caggtcccgt tc                                                22

<210> 18
<211> 20
<212> DNA
<213> Artificial Sequence

<220>
<223> Description of Artificial Sequence: synthetic DNA

<400> 18
tacatcttgg actcctcctc                                                   20

<210> 19
<211> 20
<212> DNA
<213> Artificial Sequence

<220>
<223> Description of Artificial Sequence: synthetic DNA

<400> 19
tagggattcc aagttgcctg                                                   20

<210> 20
<211> 20
<212> DNA
<213> Artificial Sequence

<220>
<223> Description of Artificial Sequence: synthetic DNA

<400> 20
ctttggtatc gtggaaggac                                                    20

<210> 21
<211> 20
<212> DNA
<213> Artificial Sequence

<220>
<223> Description of Artificial Sequence: synthetic DNA

<400> 21
tgacaaagtg gtcgttgagg                                                    20

<210> 22
<211> 20
<212> DNA
<213> Artificial Sequence

<220>
<223> Description of Artificial Sequence: synthetic DNA

<400> 22
tgaagtaccc catcgagcac                                                    20

<210> 23
<211> 20
<212> DNA
<213> Artificial Sequence

<220>
<223> Description of Artificial Sequence: synthetic DNA

<400> 23
tctcttgctc gaagtccagg                                                    20